Europäisches Patentamt

**European Patent Office**

Office européen des brevets

(19)

(11) Numéro de publication : **0 079 832**
**B1**

(12)

# FASCICULE DE BREVET EUROPÉEN

(45) Date de publication .du fascicule du brevet :
**30.07.86**

(21) Numéro de dépôt : **82402071.3**

(22) Date de dépôt : **12.11.82**

(51) Int. Cl.⁴ : **C 12 M   1/00, E 21 B 43/00**

(54) **Procédé et dispositif de fabrication de gaz combustible par digestion anaérobie de résidus organiques.**

(30) Priorité : **16.11.81 FR 8121356**

(43) Date de publication de la demande :
**25.05.83 Bulletin 83/21**

(45) Mention de la délivrance du brevet :
**30.07.86 Bulletin 86/31**

(84) Etats contractants désignés :
**BE DE GB IT**

(56) Documents cités :
**FR-A- 2 374 413**
**FR-A- 2 388 986**
**FR-A- 2 482 411**
**US-A- 3 724 542**
**US-A- 3 826 308**
**US-A- 4 085 972**
**US-A- 4 273 615**
**US-A- 4 276 778**
**CHEMICAL & ENGINEERING NEWS, vol.56, no.35, 28
août 1978, NEW YORK (US), "Landfill sites eyed as
methane source"**

(73) Titulaire : **S.I.C.A. PULPOSEC DE CHEVRIERES
68 Boulevard des Etats Unis
F-60204 Compiègne Cedex (FR)**

(72) Inventeur : **Fontan, Gérard
14 rue de la Croix
F-95300 Ennery (FR)**
Inventeur : **Cambon, Jean-Luc
16 Place du Marché
F-92200 Neuilly sur Seine (FR)**

(74) Mandataire : **Pinguet, André
CAPRI 28 bis, avenue Mozart
F-75016 Paris (FR)**

## Description

La présente invention a trait à un procédé et à un dispositif de fabrication de gaz combustible par fermentation d'un milieu constitué par des éléments végétaux en suspension dans l'eau. L'invention s'applique notamment à la production de méthane par fermentation, ou digestion de déchets tels que la pulpe de betterave, résidu de la fabrication de sucre, mais peut s'appliquer à d'autres produits tels que le topinambour, les déchets de canne à sucre et autres produits organiques végétaux, résidus ou non. L'invention englobe aussi la digestion (fermentation méthanique) anaérobie des eaux usées à forte charge en DBO (Demande Biologique d'Oxygène).

Dans un ordre d'idées voisin, on a déjà proposé de faire fermenter des déchets, tels que des eaux d'égout, pour obtenir du méthane. On utilise des hydrocarbures ou des schistes bitumineux comme source alimentaire pour les micro-organismes de la fermentation. Pour ce faire, on a utilisé comme volumes de travail, des puits dans des gisements d'hydrocarbures épuisés. L'application d'un tel procédé est limitée géographiquement aux régions pétrolières.

Conformément à la présente invention, la fermentation est effectuée dans une cavité souterraine, existante ou réalisée à cet effet, formée dans une roche imperméable ou une roche perméable saturée d'eau, en dessous du niveau de la nappe phréatique, la pression du milieu en réaction étant inférieure à la pression hydrostatique de l'eau dans le massif perméable. Avantageusement, la température est maintenue entre 30 et 40° de préférence au voisinage de 35 °C.

On peut ainsi réaliser des volumes de réaction de très grandes dimensions, pouvant soutenir des pressions élevées, à des prix de revient beaucoup plus bas qu'avec de la chaudronnerie classique. Une application importante est constituée par la digestion de la pulpe de betterave produite dans les sucreries. La digestion de la pulpe permet d'une part d'éliminer toute ou partie de celle-ci et d'autre part de produire un gaz combustible qui peut fournir une part importante de la consommation d'énergie de la sucrerie, par exemple de l'ordre de la moitié. En général les cultures de betteraves se font dans des régions où la roche peut convenir pour la réalisation de cavités souterraines. Non seulement le prix de revient au mètre cube d'installation est compétitif, mais l'entretien de la cavité est peu coûteux, elle est peu sensible aux accidents ou aux tentatives de destruction, quelle qu'en soit l'origine, et écologiquement, une telle installation ne modifie pas le paysage.

Le gaz obtenu dans une telle fermentation est souvent dénommé Biogaz, il contient en moyenne 60 % de méthane et 40 % de gaz carbonique. Le processus de fermentation est connu, il est inutile de le décrire ici.

Les déchets agricoles, notamment les pulpes de betterave à sucre sortent de la sucrerie en suspension dans l'eau et une concentration favorable pour la digestion dans une cavité correspond à une teneur en matière sèche de l'ordre de 4 à 10 %. Cette concentration est obtenue facilement, en enlevant ou en ajoutant de l'eau aux produits sortant de la sucrerie.

La pression est un grand avantage car le gaz étant produit sous pression, cela permet un stockage tampon très commode. En situant la cavité à une profondeur de l'ordre de 50 à 100 m, on peut ainsi faire fonctionner la fermentation sous une pression de 0 à 6 bars sans aucune difficulté. Dans une roche perméable on maintient la pression de travail inférieure à la pression hydrostatique de façon à éviter la fuite de liquide ou de gaz dans le massif ainsi que la migration des bactéries dans la roche. (Les bactéries ne peuvent pas aller, à contre courant, dans un milieu froid et sans nourriture). On aura donc un courant d'eau ruisselant sur la surface de la cavité, eau qui vient s'ajouter au liquide en réaction, et qui sera extraite en même temps que le jus épuisé, et en constitue toujours une très faible proportion. Le débit d'eau de ruissellement dépend de l'écart entre la pression de travail dans la cavité, et la pression hydrostatique dans le massif. On comprendra que plus l'écart est grand, plus le débit de ruissellement est important. De toute façon, ce débit est du deuxième ordre par rapport au débit de jus fermentescible dans la cavité.

Le volume de la cavité dépend du débit de résidus à traiter et du temps de séjour du jus dans la cavité.

Un temps de séjour approprié est compris entre cinq et dix jours environ. La digestion pourra être suffisamment poussée et l'eau résiduaire sera ainsi rejetée sans traitement de finition. Si la digestion n'est pas complète les matières sèches résiduelles pourront être utilisées comme amendements, engrais, ou aliments pour animaux, ou encore pourront être réintroduits dans la cavité. La quantité de gaz produite dépend des conditions de la réaction et du degré d'avancement de l'extraction auquel on désire parvenir compte tenu de l'utilisation éventuelle souhaitée pour le résidu. En moyenne, on pourra obtenir par jour un volume de gaz (ramené à 1 atmosphère) correspondant au volume de la cavité.

Le volume de la cavité pourra être limité par le souci d'avoir des dimensions acceptables. La section est limitée par la nature de la roche et les caractéristiques mécaniques du terrain (état de contraintes, fissurations, etc.). On pourra éventuellement former deux cavités ou plus pour une sucrerie importante en communication, ou indépendante. En général, les pulpes de betterave d'une sucrerie moyenne pourront être traîtées dans un volume de l'ordre de 50 000 mètres cube.

La profondeur de la cavité sera choisie en fonction de la pression de fonctionnement désirée pour le gaz, et en fonction de la position de la nappe phréatique, de façon à avoir une pression hydrostatique supérieure à la pression maximale de service dans la cavité, et en tenant compte de la forme de la cavité (voir notamment brevet français n° 80 00546 du 11/01/1980 FR-A-24 73 618).

La partie supérieure de la cavité sera occupée par le gaz, qui pourra représenter 10 à 20 % du volume total. Ce volume tampon dépend des conditions d'utilisation du gaz. Dans le cas d'une sucrerie, le fonctionnement est permanent pendant environ cent jours, et la consommation de gaz est pratiquement constante. La variation du volume de stockage est obtenue par action sur le pompage d'extraction du jus digéré. En variante, le volume de gaz peut être plus important et servir de stockage.

Bien que la température préférable soit comprise entre 30 et 40 °C, il est possible de conduire de réaction dans une autre plage de températures, autour de 55 °C. Mais d'une part, la réaction est plus sensible aux écarts de températures dans cette plage, et en outre, comme les parois de la cavité sont à la température géothermique, souvent environ 15 °C, on a dans les zones marginales un gradient de température, qui est beaucoup moins favorable pour une réaction pilotée à 55 °C qu'à 35 °C.

Un brassage peut être utile ou même nécessaire, selon la forme et la dimension de la cavité. Il sera avantageusement obtenu par barbotage du gaz recueilli, refoulé par un compresseur dans un tube perforé.

L'amorçage de la digestion peut être obtenu simplement par introduction de boues activées judicieusement choisies.

D'autres caractéristiques de l'invention apparaîtront au cours de la description qui va suivre, donnée à titre d'exemple non limitatif, en regard des dessins ci-joints et qui fera bien comprendre comment l'invention peut être réalisée.

Les dessins montrent ;

figure 1 une coupe longitudinale d'une cavité de fermentation selon l'invention, et

figures 2 et 3 des vues en plan schématique de formes possibles de cavités.

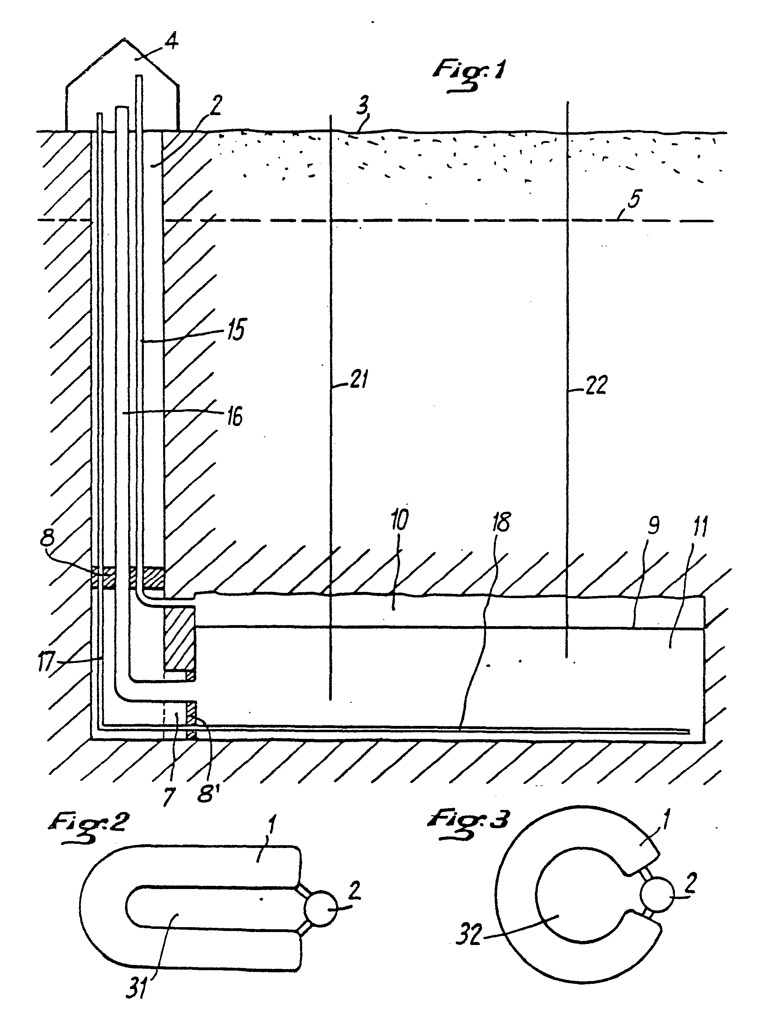

Sur la figure 1, la cavité 1 est représentée rectiligne, mais elle peut avoir toute forme, comme par exemple représenté sur les figures 2 et 3. On peut supposer que la coupe est effectuée par une surface verticale suivant la ligne médiane de la cavité. Un puits 2 est situé au voisinage d'une extrémité de la cavité. Il sert à l'extraction des déblais pendant le creusement de la cavité, et au passage de diverses canalisations utiles à la marche de l'installation. Le puits peut déboucher, à la surface 3 du sol dans une construction 4 abritant les têtes de tubes, la robinetterie, les accessoires de régulation, etc... De tels moyens sont bien connus. Il existe un choix très large chez divers fabricants et il n'est pas nécessaire de les décrire plus en détail ici. On a représenté en 5 le niveau de la nappe phréatique. Le puits 2 est isolé de la cavité par un bouchon horizontal 8 ou vertical 8' traversé par les divers tubes et communique avec la cavité 1 par une galerie 7, aussi courte que possible. On a représenté sur la figure 1 un bouchon horizontal 8 et un bouchon vertical 8', mais bien entendu il n'y en a qu'un. On a représenté en 9 le niveau de séparation entre le ciel gazeux 10 et le jus 11 en fermentation. Le puits abrite une conduite de sortie 15 du gaz, au moins un tube 16 de passage de jus ; l'entrée et la sortie peuvent être situées dans le même puits ou dans des puits différents. Seul un tube est représenté pour simplifier le dessin. La position du deuxième tube dépend de la forme de la cavité comme on l'expliquera ci-après. A titre d'exemple, les tubes d'introduction et d'extraction du jus peuvent avoir un diamètre de l'ordre de 15 cm. Le tube de sortie du gaz peut avoir une dimension du même ordre. Comme le jus en digestion se déplace d'une extrémité de la cavité vers l'autre extrémité, il est possible de n'avoir qu'un seul puits 2 si les deux extrémités de la cavité sont proches l'une de l'autre (figures 2 et 3).

Le puits 2 abrite encore une descente 17 de gaz sous pression, pour alimenter une rampe 18 de dégagement de gaz en vue du brassage. L'installation peut comporter des capteurs de pression et plusieurs sondes thermométriques 21, 22, etc, donnant les températures en différents points de la cavité, en différentes profondeurs et à différentes hauteurs. Les formes des figures 2 et 3 sont favorables thermiquement : les massifs centraux 31 (figure 2) et 32 (figure 3) prenant une température supérieure. La forme de la figure 3 est plus favorable du point de vue de la circulation. On doit en effet éviter les zones sans mouvement où se forme une stagnation.

**Revendications**

1. Procédé de fabrication de gaz combustible par fermentation d'un milieu constitué d'éléments végétaux en suspension dans l'eau, caractérisé en ce que la fermentation est conduite dans une cavité souterraine (1), formée dans une roche imperméable ou dans une roche perméable saturée d'eau, en dessous du niveau (5) de la nappe phréatique, la pression du milieu en réaction étant inférieure à la pression hydrostatique de l'eau dans le massif perméable.

2. Procédé selon la revendication 1, caractérisé en ce que la pression du milieu en fermentation dans la cavité est comprise entre 0 et 6 bars au-dessus de l'atmosphère.

3. Procédé selon une des revendications 1 ou 2, caractérisé en ce que la température est maintenue entre 30 et 40 °C, de préférence au voisinage de 35 °C.

4. Procédé selon une des revendications précédentes, caractérisé en ce que le temps de séjour du milieu en fermentation est compris entre 5 et 10 jours.

5. Procédé selon une des revendications précédentes, caractérisé en ce que le milieu en fermentation est agité par dégagement, en fond de cavité de bulle du gaz produit par fermentation et renvoyé par un compresseur.

6. Procédé selon une des revendications précédentes, caractérisé en ce que l'on maintient dans la cavité un ciel gazeux (10) occupant 10 à 20 % du volume total de la cavité.

7. Procédé selon la revendication 1, caractérisé en ce que l'on utilise la cavité pour stocker le gaz produit.

8. Cavité souterraine (1) pour la mise en œuvre du procédé selon une des revendications précédentes, caractérisée en ce qu'elle a une longueur horizontale grande par rapport à la section, qu'elle a une forme non rectiligne suivant sa longueur, et que les extrémités sont voisines l'une de l'autre, et qu'un puits vertical (2) relie l'extérieur à une zone située au voisinage des deux extrémités voisines de la cavité.

9. Cavité souterraine selon la revendication 8, caractérisée en ce qu'elle comporte le long du fond, une rampe (18) de distribution de gaz constituée par un tube perforé, connecté à un compresseur.

## Claims

1. Method for producing combustible gas by fermenting a medium consisting of plant matter suspended in water, characterised in that fermentation is performed in an underground cavity (1), which is formed in an impervious rock or in a pervious rock saturated with water, below the level (5) of the groundwater table, the pressure of the reacting medium being less than the hydrostatic pressure of the water in the pervious mass.

2. Method according to claim 1, characterised in that the pressure of the fermenting medium in the cavity is between 0 and 6 bars above atmospheric.

3. Method according to either one of claims 1 or 2, characterised in that the temperature is maintained between 30 and 40 °C and preferably in the vicinity of 35 °C.

4. Method according to one of the preceding claims, characterised in that the residence time of the fermenting medium is between 5 and 10 days.

5. Method according to one of the preceding claims, characterised in that the fermenting medium is agitated by releasing, at the bottom of the cavity, gas bubbles produced by means of fermentation and returned via a compressor.

6. Method according to one of the preceding claims, characterised in that a gaseous headspace (10) occupying 10 to 20 % of the total volume of the cavity is maintained inside the cavity.

7. Method according to claim 1, characterised in that the cavity is used to store the gas produced.

8. Underground cavity (1) for implementing the method according to one of the preceding claims, characterised in that it has a large horizontal length in relation to the cross-section, in that it has a nonrectilinear shape along its length, in that the ends are adjacent to each other and in that a vertical well (2) connects the outside to a zone situated in the vicinity of the two adjacent ends of the cavity.

9. Underground cavity according to claim 8, characterised in that it has along the bottom, a gas distribution bar (18) consisting of a perforated tube connected to a compressor.

## Patentansprüche

1. Verfahren zur Erzeugung von brennbarem Gas mittels Fermentation eines Stoffes, der aus in Wasser suspendierten Pflanzenteilen besteht, dadurch gekennzeichnet, daß die Fermentation in einem unterirdischen Raum (1) durchgeführt wird, der in einem wasserdichten Felsen oder in einem wasserdurchlässigen, mit Wasser gesättigtem Felsen unterhalb des Grundwasserspiegels (5) ausgebildet ist, wobei der Druck des reagierenden Stoffes kleiner ist als der hydrostatische Druck des Wassers im wasserdurchlässigen Gestein.

2. Verfahren nach Anspruch 1, dadurch gekennzeichnet, daß der Druck des fermentierenden Stoffes im Raum zwischen 0 und 6 bar oberhalb des Atmosphärendrucks beträgt.

3. Verfahren nach einem der Ansprüche 1 oder 2, dadurch gekennzeichnet, daß eine Temperatur zwischen 30 und 40 °C aufrechterhalten wird, vorzugsweise in der Nähe von 35 °C.

4. Verfahren nach einem der vorhergehenden Ansprüche, dadurch gekennzeichnet, daß die Fermentationszeit des Stoffes zwischen 5 und 10 Tagen beträgt.

5. Verfahren nach einem der vorhergehenden Ansprüche, dadurch gekennzeichnet, daß der fermentierende Stoff durch Gasblasen bewegt wird, die am Boden des Raums durch Fermentation entstehen und durch einen Kompressor rückgeleitet werden.

6. Verfahren nach einem der vorhergehenden Ansprüche, dadurch gekennzeichnet, daß in dem Raum eine Gasdeckschicht (10) aufrechterhalten wird, die 10 bis 20 % des Gesamtvolumens des Raums einnimmt.

7. Verfahren nach Anspruch 1, dadurch gekennzeichnet, daß der Raum zur Speicherung des erzeugten Gases verwendet wird.

8. Unterirdischer Raum (1) zur Durchführung des Verfahrens nach einem der vorhergehenden Ansprüche, dadurch gekennzeichnet, daß er eine große waagrechte Länge bezüglich seines Querschnitts aufweist, daß seine Gestalt in Längsrichtung nicht geradlinig ist und daß seine Enden zueinander benachbart sind und daß ein senkrechter Schacht (2) das Äußere mit einem Bereich in der Nähe der beiden benachbarten Enden des Raumes verbindet.

9. Unterirdischer Raum nach Anspruch 8, dadurch gekennzeichnet, daß er entlang seines Bodens eine Gasverteilungsleitung (18) aufweist, die aus einem perforierten Rohr besteht, das mit einem Kompressor verbunden ist.

*Fig.1*

*Fig.2*

*Fig.3*